(19) Europäisches Patentamt
European Patent Office
Office européen des brevets

(11) Veröffentlichungsnummer : **0 494 455 B1**

(12) **EUROPÄISCHE PATENTSCHRIFT**

(45) Veröffentlichungstag der Patentschrift :
**27.07.94 Patentblatt 94/30**

(51) Int. Cl.$^5$ : **C07C 209/68,** C07C 211/54

(21) Anmeldenummer : **91122363.4**

(22) Anmeldetag : **30.12.91**

# (54) Verfahren zur Herstellung von Diphenylaminen.

(30) Priorität : **10.01.91 DE 4100514**
**16.01.91 DE 4101066**

(43) Veröffentlichungstag der Anmeldung :
**15.07.92 Patentblatt 92/29**

(45) Bekanntmachung des Hinweises auf die Patenterteilung :
**27.07.94 Patentblatt 94/30**

(84) Benannte Vertragsstaaten :
**DE FR GB**

(56) Entgegenhaltungen :
**EP-A- 0 208 933**
**EP-A- 0 325 132**
**DE-A- 2 331 878**
**DE-A- 2 520 893**

(73) Patentinhaber : **BAYER AG**
**D-51368 Leverkusen (DE)**

(72) Erfinder : **Immel, Otto, Dr.**
**Immenhofweg 26**
**W-4150 Krefeld (DE)**
Erfinder : **Waldmann, Helmut, Dr.**
**Henry.Th.-v.-Böttinger-Strasse 15**
**W-5090 Leverkusen 1 (DE)**
Erfinder : **Langer, Reinhard, Dr.**
**Scheiblerstrasse 111**
**W-4150 Krefeld (DE)**
Erfinder : **Darsow, Gerhard, Dr.**
**Zu den Tannen 39**
**W-4150 Krefeld (DE)**
Erfinder : **Buysch, Hans-Josef, Dr.**
**Brandenburger Strasse 28**
**W-4150 Krefeld (DE)**

EP 0 494 455 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft ein Verfahren zur Herstellung von Diphenylamin, das durch niederes Alkyl und/oder niederes Alkoxy substituiert sein kann, durch Dehydrierung des korrespondierenden N-Cyclohexyliden-anilins in Gegenwart eines Rhodium enthaltenden Trägerkatalysators.

Zur Herstellung von Diphenylamin und dessen Derivaten ist aus DE-OS 23 31 878 ein Verfahren bekannt, bei dem man von Iminen, wie N-Cyclohexyliden-anilin und dessen Derivaten ausgeht und sie in der Gasphase in Gegenwart von Trägerkatalysatoren auf der Basis von Nickel, Platin, Palladium oder Kupfer-Chrom dehydriert. Nachteile dieses Verfahrens sind ungenügende Selektivität und geringe Standfestigkeit der Katalysatoren, so daß die Ausbeute an Diphenylamin bereits nach wenigen Stunden stark abnimmt.

In der DE-OS 2 520 893 werden Nickel-Chrom-Misch-Katalysatoren, die als Nebenbestandteile Aluminium, Kupfer und Erdalkali- sowie Alkalisulfate enthalten, sowie Nickel-Chrom-Mischkatalysatoren mit Zusätzen von Mangan und Aluminium beschrieben. Die beschriebenen Katalysatoren erreichen nur unter Wasserstoffatmosphäre sehr hohe Standzeiten von bis zu 6.000 Stunden, wobei aber der Umsatz und die Selektivität unbefriedigend sind. In einem Inertgasträgerstrom sind die Standzeiten deutlich kürzer, wobei je nach Temperatur entweder deutliche Mengen an Cyclohexylanilin oder Carbazol als störendem Nebenprodukt neben schlecht abtrennbarem Methyldiphenylamin auftreten. Weitere Nachteile der beschriebenen Katalysatoren sind die geringe Belastbarkeit und ihre begrenzte Regenerierbarkeit, was ihren Wert für den technischen Einsatz beeinträchtigt.

In EP 208 933 werden Rhodium-Trägerkatalysatoren beschrieben, deren besonderes Merkmal die Vorbehandlung des Trägermaterials mit Chrom- und Mangansalzen ist. Diese Katalysatoren werden als geeignete Kontakte zur Gasphasendehydrierung von ganz oder teilweise hydrierten, gegebenenfalls substituierten Hydroxydiphenylen beschrieben, wobei in einer Wasserstoffatmosphäre gearbeitet wird, d.h. ohne Inertgas-Trägerstrom. Die Selektivitäten bei den in den Beispielen aufgeführten Dehydrierungen von 2-Cyclohexyliden- / 2-Cyclohexenyl-Cyclohexanon-Gemischen übersteigen im allgemeinen die 87 %-Marke nicht.

In DE-OS 38 01 754 werden Rhodium-Trägerkatalysatoren beschrieben, deren besonderes Merkmal außer der Vorbehandlung des Trägermaterials mit Chrom- und Mangansalzen die zusätzliche Dotierung mit mindestens einem Metall aus der Gruppe Palladium, Platin und Iridium ist. Diese Katalysatoren werden als geeignete Kontakte zur Gasphasendehydrierung von gegebenenfalls substituierten Dicyclohexylaminen zu den korrespondierenden Diphenylaminen beschrieben, wobei die Anwesenheit von Cyclohexylanilinen und Anilin sowie die von Ammoniak als nicht störend beschrieben wird. Als Trägergase können Wasserstoff, Stickstoff und Alkane eingesetzt werden.

In DE-OS 38 01 754 werden die bis dato beschriebenen Verfahren zur Diphenylaminsynthese, ausgehend von teilweise hydrierten, gegebenenfalls substituierten Diphenylaminen, also auch die Dehydrierung von Cyclohexyliden-anilinen, als unzureichend für eine technische Anwendung beschrieben.

Die Unzulänglichkeiten der Dehydrierung von Cyclohexyliden-anilinen sind in Verbindung mit der begrenzten chemischen Stabilität dieser Verbindungsklasse zu sehen, welche zur Selbstkondensation unter Anilinabspaltung neigen, wobei oligomere Cyclohexenylverbindungen entstehen. Vor diesem Hintergrund ist die Beschränkung der DE-OS 38 01 754 auf die sehr stabilen Dicyclohexylamine einzustufen. Da in DE-OS 38 01 754 ausschließlich Katalysatoren auf der Basis des Trägermaterials, welches in EP 208 933 beansprucht wird, beschrieben werden, mit dem Unterschied, daß außer Rhodium ein weiteres Platinmetall auf dem Trägermaterial niedergeschlagen wird, folgt, daß die Katalysatoren aus EP 208 933 für eine Diphenylaminsynthese durch Dehydrierung nur bedingt, nämlich bei Anwendung auf bestimmte Substrate, geeignet sein sollten.

Es wurde nun überraschend gefunden, daß die in EP 208 933 beschriebenen Katalysatoren besonders gut zur Gasphasendehydrierung von gegebenenfalls symmetrisch oder unsymmetrisch substituierten N-Cyclohexyliden-anilinen zu Diphenylaminen geeignet sind, wobei storende Nebenreaktionen weitgehend unterdrückt werden.

Dies ist von besonderer technischer Bedeutung, weil symmetrisch und vor allem unsymmetrisch substituierte N-Cyclohexyliden-aniline besonders leicht aus den entsprechenden Komponenten Cyclohexanon und Anilin unter Wasserabspaltung herzustellen sind.

Es wurde nun gefunden, daß man in guter Ausbeute und hoher Selektivität Diphenylamine der Formel

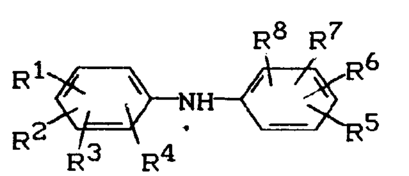

(I)

in der

| | |
|---|---|
| $R^1$ | für Wasserstoff, $C_1$-$C_6$-Alkyl, $C_1$-$C_2$-Alkoxy, $C_3$-$C_6$-Cycloalkyl, Benzyl oder Aryl steht, |
| $R^2$ | für Wasserstoff, $C_1$-$C_6$-Alkyl oder $C_1$-$C_2$-Alkoxy steht, |
| $R^5$ | Wasserstoff, $C_1$-$C_6$-Alkyl, $C_1$-$C_6$-Alkoxy, $C_3$-$C_6$-Cycloalkyl, Benzyl, Aryl, Hydroxy, Amino, $C_1$-$C_6$-Alkylamino, Di-$C_1$-$C_6$-alkylamino, Aryloxy oder Arylamino bedeutet, |
| $R^6$ | für Wasserstoff, $C_1$-$C_6$-Alkyl, $C_1$-$C_6$-Alkoxy, Hydroxy, Amino oder $C_1$-$C_6$-Alkylamino steht und |
| $R^3$, $R^4$, $R^7$ und $R^8$ | unabhängig voneinander Wasserstoff oder $C_1$-$C_2$-Alkyl bedeuten, |

wobei Aryl für Phenyl oder für in 2-, 3- oder 4-Stellung gebundenes 5- oder 6-gliedriges Heteroaryl mit 1 oder 2 Heteroatomen aus der Gruppe N, O und S steht,
dadurch erhält, daß man Cyclohexyliden-aniline der Formel

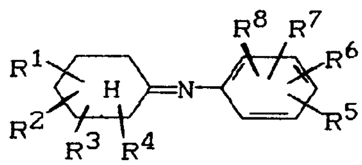

(II),

in der
die Reste $R^1$ bis $R^8$ die obige Bedeutung haben,
bei 250-500°C und einen Druck von 10 mbar bis 100 bar an einem Katalysator dehydriert, der einen Rhodiumgehalt ohne weitere Platinmetalle von 0,05-5 Gew.-%, bevorzugt 0,05-4 Gew.-% besonders bevorzugt 0,1-3 Gew.-% aufweist, und der weiterhin Zusätze von 1-6 Gew.-% eines Alkalimetallhydroxids und 1-6 Gew.-% eines Alkalimetallsulfats enthält, wobei alle Prozentsätze auf das Gesamtgewicht des Katalysators bezogen sind.

Als Alkylreste seien Methyl, Ethyl, Propyl, Isopropyl, Butyl, Isobutyl, tert.-Butyl und Hexyl, bevorzugt Methyl und Ethyl genannt.

Als Alkoxyreste seien Methoxy, Ethoxy, Propoxy, Isopropoxy, Butoxy, Isobutoxy, tert.-Butoxy und Hexyloxy, bevorzugt Methoxy und Ethoxy genannt.

In den Alkyl-, Alkoxy- und Alkyl(Dialkyl)aminoresten kann der Kohlenwasserstoffteil geradkettig oder verzweigt sein.

Als einkernige isocyclische oder heterocyclische Arylreste seien Furanyl, Pyrrolyl, Pyridyl und Phenyl, bevorzugt Phenyl genannt.

Durch Kondensation von gleich oder verschieden substituierten Anilinen und Cyclohexanonen erhält man symmetrisch oder unsymmetrisch substituierte N-Cyclohexyliden-aniline, beispielsweise N-Cyclohexyliden-anilin, N-Cyclohexyliden-2-methyl-anilin, N-Cyclohexyliden-4-phenyl-anilin und N-2-methylcyclohexyliden-anilin; insbesondere sei N-Cyclohexylidenanilin erwähnt.

Unter den Cyclohexyliden-anilinen (II) kommen für das erfindungsgemäße Verfahren diejenigen in Betracht, die im Bereich der Reaktionstemperatur, beispielsweise bei 300°C, einen Dampfdruck von mindestens 5 mbar, bevorzugt mindestens 10 mbar, besonders bevorzugt mindestens 20 mbar haben.

In bevorzugter Weise werden Cyclohexyliden-aniline eingesetzt, in denen Phenyl für Aryl steht. In weiterhin bevorzugter Weise werden Cyclohexyliden-aniline eingesetzt, in denen $R^4$ und $R^8$ Wasserstoff bedeuten, besonders bevorzugt solche, in denen $R^3$, $R^4$, $R^7$ und $R^8$ Wasserstoff bedeuten.

Bevorzugte Cyclohexyliden-aniline sind weiterhin solche der Formel

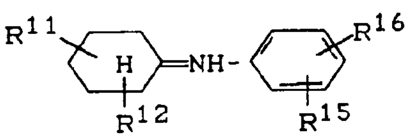

(III),

in der

| | |
|---|---|
| $R^{11}$ | Wasserstoff, $C_1$-$C_6$-Alkyl, $C_1$-$C_2$-Alkoxy, Cyclopropyl, Cyclopentyl, Cyclohexyl oder Phenyl bedeutet, |
| $R^{12}$ | für Wasserstoff, $C_1$-$C_2$-Alkyl oder $C_1$-$C_2$-Alkoxy steht, |
| $R^{15}$ | Wasserstoff, $C_1$-$C_6$-Alkyl, $C_1$-$C_4$-Alkoxy, Cyclopropyl, Cyclopentyl, Cyclohexyl, Phenyl, Hydroxy, Amino, $C_1$-$C_6$-Alkylamino, Di-$C_1$-$C_4$-alkyl-amino, Phenoxy oder Phenylamino bedeutet und |

$R^{16}$ für Wasserstoff, $C_1$-$C_2$-Alkyl oder $C_1$-$C_2$-Alkoxy steht.

Besonders bevorzugte Cyclohexyliden-aniline sind solche der Formel

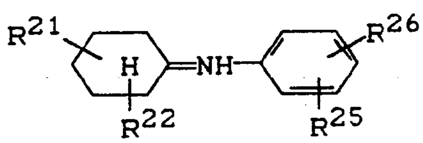

(IV),

in der

$R^{21}$, $R^{22}$ und $R^{26}$ unabhängig voneinander Wasserstoff, Methyl oder Ethyl bedeuten und

$R^{25}$ Wasserstoff, Methyl, Ethyl, Methoxy, Ethoxy, $C_1$-$C_6$-Alkylamino oder Di-$C_1$-$C_2$-alkylamino darstellen.

Als Trägermaterialien kommen die üblichen Katalysatorträger in Frage, wie α- und γ-Aluminiumoxide, Aluminiumspinelle der Zusammensetzung $Me(II)Al_2O_4$ oder $Me(I)AlO_2$, worin Me(II) ein zweiwertiges Kation des Eisens, Zinks, Nickels, Kupfers, Kobalts, Cadmiums, Magnesiums oder anderer, bevorzugt des Magnesiums, und Me(I) ein einwertiges Kation, beispielsweise des Lithiums (Li-Al-Spinell), ist, Kieselgur, Aktivkohle, Bentonit, Silicagel, Montmorillonite, $ZrO_2$, $TiO_2$, ZnO, MgO, Seltenerdoxide und Bimssteine, bevorzugt die genannten Aluminiumoxide und Aluminiumspinelle, besonders bevorzugt γ-$Al_2O_3$. Solche Träger haben eine große spezifische Oberfläche (> 50 $m^2$, bevorzugt > 100 $m^2/g$). Die Träger weisen in einer besonders bevorzugten Weise einen Gehalt an Chrom und Mangan von zusammen etwa 0,05-8 Gew.-%, bevorzugt 0,2-5 Gew.-%, bezogen auf das Gesamtgewicht des Katalysators, auf. Das Gewichtsverhältnis von Chrom und Mangan beträgt etwa 5:1-1:5, bevorzugt 2:1-1:2. Solche mit Chrom und Mangan behandelten Träger sind aus DE-OS 38 01 754 und EP 208 933 bekannt. Die Herstellung und Aktivierung dieser Katalysatoren sind daher dem Fachmann bekannt.

Der erfindungsgemäß einzusetzende Katalysator enthält Rhodium allein ohne weitere Platinmetalle. Das Rhodium liegt in einer Gesamtmenge von 0,05-5 Gew.-%, bevorzugt 0,05-4 Gew.-%, besonders bevorzugt 0,1-3 Gew,-% vor, bezogen auf das Gesamtgewicht des Katalysators.

Der einzusetzende Katalysator enthält weiterhin 1-6 Gew.-%, bevorzugt 2-5 Gew.-%, bezogen auf das Gesamtgewicht des Katalysators eines Alkalihydroxids, wie Lithiumhydroxid, Natriumhydroxid, Kaliumhydroxid, Rubidiumhydroxid, Caesiumhydroxid, bevorzugt Lithiumhydroxid, Natriumhydroxid, Kaliumhydroxid, besonders bevorzugt Natriumhydroxid oder Kaliumhydroxid. Der einzusetzende Katalysator enthält weiterhin in Kombination mit einem oder mehreren der genannten Alkalihydroxide zusätzlich 1-6 Gew.-%, bevorzugt 2-5 Gew.-%, bezogen auf das Gesamtgewicht des Katalysators, eines Alkalimetallsulfats, wie Lithiumsulfat, Natriumsulfat, Kaliumsulfat, Rubidiumsulfat, Caesiumsulfat, bevorzugt Lithiumsulfat, Natriumsulfat, Kaliumsulfat, besonders bevorzugt Natriumsulfat oder Kaliumsulfat.

Zur Herstellung der beschriebenen Katalysatoren kann in der besonders bevorzugten Weise so vorgegangen werden, daß auf ein $Al_2O_3$ oder einen Aluminium-Spinell in Form von Strangpreßlingen, Pillen oder Kugeln mit Abmessungen von etwa 2-10 mm Verbindungen des Chroms und Mangans aufgebracht werden, der so beaufschlagte Träger auf höhere Temperatur erhitzt wird, anschließend getrennt das Rhodium und eines oder mehrere Alkalihydroxide und eines oder mehrere Alkalisulfate aufgetragen werden; nach jedem Auftragen wird eine Trocknung vorgenommen, im allgemeinen bei 100-140°C bei vermindertem bis normalem Druck, wie 1-1000 mbar, bevorzugt 10-500 mbar, beispielsweise bei Wasserstrahlvakuum.

Das Aufbringen des Chroms und Mangans auf den Katalysatorträger in der besonders bevorzugten Art kann beispielsweise durch gemeinsames Ausfällen eines Mangan-Chrom-Hydroxidgemisches aus einer Chrom- und Mangansalz-Lösung mit Alkalilauge oder Ammoniak und anschließendes Auswaschen der löslichen Anteile mit Wasser erfolgen. Als Chrom- und Mangansalze kommen insbesondere die Sulfate, Chloride, Acetate und/oder Nitrate der genannten Elemente in Betracht. Die Abscheidung des Chroms und Mangans auf den Katalysatorträger kann auch als Ammonium-Mangan-Chromat oder Ammonium-Alkali-Mangan-Chromat aus einer Lösung von Mangan(II)-Salzen und Ammoniumbichromat mittels Ammoniak und/oder basischer Alkaliverbindungen erfolgen. Besonders gleichmäßige und festhaftende Abscheidungen erhält man, wenn die Basenzugabe langsam und gleichmäßig unter Vermeidung größerer Konzentrationsunterschiede erfolgt. Hierzu kann beispielsweise die Fällung mittels Harnstoff unter hydrolysierenden Bedingungen vorgenommen werden, wodurch die Bedingungen der langsamen Basenzugabe besonders gut gewährleistet sind.

Nach dem Aufbringen der Chrom- und Manganverbindungen und der beschriebenen Ausfällung kann der so beaufschlagte Katalysatorträger frei von löslichen Verbindungen gewaschen werden, bevor er auf höhere Temperaturen (etwa 200-450°C, bevorzugt 250-350°C) erhitzt wird, Nach dieser Temperung ist der mit Chrom

und Mangan beaufschlagte Träger bereit zur Tränkung mit den übrigen genannten Katalysatorbestandteilen.

Die Tränkung des Trägers mit den Rhodium bzw. mit Alkalihydroxid und Alkalisulfat (jeweils eines oder mehrere von diesen) erfolgt getrennt. Hierbei kann so vorgegangen werden, daß zunächst das Rhodium, beispielsweise in Form wäßriger Lösungen des Chlorids, Nitrats, Acetats oder anderer geeigneter Salze auf den Träger aufgetränkt werden, wobei nach einer Trocknung eine weitere Tränkung mit einer Alkalihydroxidlösung und einer Alkalimetallsulfatlösung erfolgt. Bei dieser Behandlung wird das Rhodium in Form seines Oxids oder Hydroxids ausgefällt. Das Auftränken des oder der Alkalihydroxide und des oder der Alkalimetallsulfate kann getrennt oder gemeinsam erfolgen. Nach einer abschließenden Trocknung steht der Katalysator zur Verfügung. Er wird in bevorzugter Weise im Reaktor vor seinem Einsatz durch Behandlung mit Wasserstoff bei erhöhter Temperatur, wie bei 120-450°C, bevorzugt bei 200-420°C, aktiviert.

Man kann jedoch auch den Träger zunächst mit einer Alkalihydroxidlösung tränken, anschließend trocknen und auf den so vorbehandelten basisch eingestellten Katalysatorträger die genannten Salze des Rhodiums auftragen, wobei im Moment der Tränkung auch die Ausfällung der Edelmetalle in Form ihrer Oxide oder Hydroxide erfolgt. Bei dieser Variante kann das zusätzliche Auftränken eines oder mehrerer Alkalimetallsulfate gemeinsam mit dem Alkalihydroxid, vor oder nach dem Auftragen des Alkalihydroxids oder als abschließende Tränkung nach dem Auftragen der Edelmetalle erfolgen. Auch hier erfolgt nach jedem Auftränken ein separates Trocknen. Nach der abschließenden Trocknung ist auch nach dieser Variante der Katalysator einsatzbereit und kann in der beschriebenen Weise vorab mit Wasserstoff bei erhöhter Temperatur aktiviert werden.

Anstatt den genannten Träger zur Beaufschlagung mit den genannten Stoffen zu tränken, kann er auch mit geeigneten Lösungen besprüht werden. Die hierzu erforderlichen Arbeitsgeräte und die Einstellung der gewünschten Beaufschlagung durch Wahl der Menge und Konzentration der Lösungen der genannten Elemente sind dem Fachmann im Prinzip bekannt.

Neben wäßrigen Lösungen kommen grundsätzlich auch alkoholische Lösungen oder Lösungen in niederen Carbonsäuren oder niederen Aminen in Frage, sofern die vorgesehenen Salze der Edelmetalle bzw. die basischen Alkalimetallverbindungen darin löslich sind,

Das erfindungsgemäße Verfahren wird bei einer Temperatur von 250-500°C, bevorzugt 280-450°C, besonders bevorzugt 300-425°C und einem Druck von 10 mbar bis 100 bar, bevorzugt 100 mbar bis 6 bar, besonders bevorzugt 0,9-1,2 bar, in der Gasphase durchgeführt. In einer dem Fachmann bekannten Weise werden im allgemeinen niedrigere Temperaturen im genannten Bereich niedrigeren Drücken des ebenfalls offenbarten Bereiches zugeordnet und umgekehrt.

Das umzusetzende, gegebenenfalls substituierte N-Cyclohexyliden-anilin kann selbstverständlich als solches erfindungsgemäß eingesetzt werden. Es ist jedoch von besonderem Vorteil, daß das N-Cyclohexyliden-anilin auch im Gemisch mit anderen Stoffen eingesetzt werden kann. Solche anderen Stoffe sind beispielsweise zusätzlich vorhandenes, gegebenenfalls substituiertes Anilin, das etwa aus der Spaltung des N-Cyclohexyliden-anilins hervorgegangen sein kann, oder ein Gemisch von zusätzlich vorliegendem, gegebenenfalls substituiertem Anilin und entsprechend substituiertem N-Cyclohexylanilin.

Das N-Cyclohexyliden-anilin oder sein Gemisch mit einem oder mehreren der genannten Stoffe wird vorteilhaft mit Hilfe eines Trägergasstroms an den Rhodium-Katalysator gebracht. Solche Trägergase sind beispielsweise Stickstoff, Wasserstoff, Helium, Argon, niedrige Kohlenwasserstoffe, wie Methan, Ethan oder Erdgas und andere oder Gemische aus diesen Trägergasen. In bevorzugter Weise werden Stickstoff, Methan, Erdgas oder Wasserstoff oder ein Gemisch von ihnen als Trägergas eingesetzt. In besonders bevorzugter Weise wird Stickstoff oder Erdgas eingesetzt. Das Trägergas wird in einer Menge von 1-100 l/g Ausgangsmaterial, bevorzugt 1-50 l/g Ausgangsmaterial, eingesetzt. Die Katalysatorbelastung wird mit 0,01-1 kg Ausgangsmaterial je Liter Katalysator und Stunde, bevorzugt 0,1-0,8, besonders bevorzugt 0,3-0,5, festgesetzt.

Das erfindungsgemäße Verfahren wird im allgemeinen kontinuierlich in Apparaten, wie sie dem Fachmann zur technischen Durchführung von Umsetzungen von Gasen an festen Kontakten allgemein bekannt sind, durchgeführt.

Zur Beladung des gasförmigen Verdünnungsmittels führt man dies durch einen Verdampfer für das Ausgangsmaterial. Zweckmäßigerweise benutzt man hierzu Fallfilm-bzw. Dünnschichtverdampfer gängiger Bauart.

Die Aufarbeitung des entstehenden Produktgemisches ist dem Fachmann ebenfalls prinzipiell bekannt. Anfallendes Anilin wird zweckmäßigerweise zur Synthese des Ausgangsmaterials wiederverwendet.

Der technische Fortschritt des erfindungsgemäßen Verfahrens liegt in der sehr hohen Standfestigkeit der Katalysatoren bei der Dehydrierung der N-Cyclohexyliden-aniline sowie ihrer guten und häufigen Regenerierbarkeit, im vollständigen Umsatz bei sehr guter Selektivität an Diphenylamin und der Bildung gut abtrennbarer Nebenprodukte. Damit werden gut zugängliche Ausgangsmaterialien, die symmetrisch oder unsymmetrisch substituiert sein können, einer technisch interessanten Umwandlung in einfacher Weise zugeführt.

Beispiele

Beispiel 1

200 g kugelförmiges $\gamma$-$Al_2O_3$ mit einem Durchmesser von 2-5 mm und einer spezifischen Oberfläche von 350 $m^2/g$ wurden in einem Rundkolben vorgelegt und mit einer Lösung von 16,6 g $MnSO_4 \cdot 4H_2O$, 12,4 g $(NH_4)_2Cr_2O_7$ und 90 g Harnstoff in 144 ml Wasser versetzt. Unter drehender Bewegung wurde der Kolben 1 Stunde lang auf 85°C gehalten, die nicht aufgenommene Flüssigkeit abfiltriert, der Katalysatorträger sulfatfrei gewaschen und dann innerhalb von 25 Stunden bei 110°C unter Wasserstrahlvakuum getrocknet. Anschließend wurde der so behandelte Katalysatorträger 30 Minuten bei 300°C getempert.

Der so hergestellte Katalysatorträger wurde mit einer Lösung getränkt, die aus 5,58 g $RhCl_3$ und 66 g Wasser hergestellt worden war. Nach einer Zwischentrocknung wurde der Katalysator nochmals mit einer Lösung von 5,84 g NaOH in 60 g Wasser getränkt und erneut getrocknet.

50 g des so erhaltenen Katalysators wurden mit einer Lösung von 1,5 g $K_2SO_4$ in 15 g Wasser getränkt und schließlich 18 Stunden bei 100°C getrocknet.

30 ml (29 g) des so hergestellten rhodiumhaltigen Katalysators wurden in einem senkrecht angeordneten, elektrisch beheizten Glasrohr von 17 mm Innendurchmesser und 70 cm Länge, 66 Stunden im Wasserstoffstrom (10 l/h) bei 400°C aktiviert. Zur Dehydrierung von N-Cyclohexyliden-anilin wurde die Ofentemperatur auf 360°C eingestellt und Stickstoff (10 l/h) als Trägergas verwendet. N-Cyclohexyliden-anilin wurde unter Benutzung einer geeichten Dosiervorrichtung von oben her im Gleichstrom mit dem Trägergas eingeleitet (~ 6,5 g/h). Im oberen Teil des Reaktionsrohres, in dem sich nur Füllkörper befanden, verdampfte das N-Cyclohexyliden-anilin und strömte zusammen mit dem Trägergas durch die Katalysatorschicht, die sich im mittleren Abschnitt des Reaktionsrohres befand. Das entstandene Reaktionsprodukt wurde auskondensiert und gaschromatographisch analysiert.

Bei einer Katalysatorbelastung von 0,13-0,3 g N-Cyclohexyliden-anilin / ml · h zeigte das Reaktionsprodukt folgende Zusammensetzung in Abhängigkeit von der Betriebsdauer des Katalysators (Rest zu 100 % sind Nebenprodukte):

| Betriebsdauer (h) | 42 | 64 | 407 | 471 |
|---|---|---|---|---|
| Diphenylamin: | 86,1% | 94,0% | 94,4% | 94,3% |
| N-Cyclohexyl-anilin: | - | - | 0,15% | 0,4% |
| Anilin: | 11,8% | 3,3% | 3,5% | 3,4% |

Beispiel 2

2,3 l des in EP 208 933, Beispiel 5, beschriebenen Katalysators wurden in ein durch Widerstandsheizung thermostatisiertes Rohr von 2,5 m Länge und 4 cm Durchmesser gefüllt. Die Länge der Katalysatorschüttung betrug 156 cm. Der Katalysator wurde 12 Stunden in einem trockenen Stickstoffstrom bei 200°C getrocknet und 24 Stunden in einem Wasserstoffstrom bei 300°C behandelt.

Das Reaktorrohr war mit einem 2 m langen Umlauffallfilmverdampfer verbunden, durch den ein trockener Stickstoffstrom von 600 l Stickstoff pro Stunde geleitet wurde. Im Umlauf wurden 2.000 ml N-Cyclohexyliden-anilin durch den Fallfilmverdampfer gepumpt, wobei die Temperatur im Inneren des Verdampfers langsam heraufgesetzt wurde, bis 700 g Edukt pro Stunde verdampften. Das eingespeiste Edukt bestand zu 99 % aus N-Cyclohexyliden-anilin und zu 1 % aus Anilin. Der Temperaturgradient im Rohrreaktor wurde so eingestellt, daß die minimale Temperatur 350°C nicht unterschritt und die maximale Temperatur 410°C nicht überstieg. Kondensierte man das Reaktionsprodukt und analysierte es gaschromatographisch, so zeigte es die folgende Zeitabhängigkeit in der Zusammensetzung:

| Betriebsstunden | 100 | 200 | 1000 | 2000 |
|---|---|---|---|---|
| Diphenylamin | 95,9 | 96,5 | 97,0 | 95,5 |
| N-Cyclohexyl-anilin | 0,15 | 0,07 | 0,05 | 0,15 |
| N-Cyclohexyliden-anilin | 0,15 | - | - | 0,24 |
| Anilin | 2,1 | 2,0 | 1,55 | 1,9 |
| Benzol | 1,1 | 0,9 | 0,8 | 1,5 |
| Hochsieder *) | 0,6 | 0,6 | 0,6 | 0,7 |

*) Carbazol, 2-Phenyl-diphenylamin, Phenylcarbazol

Der Katalysator wurde nach 2.000 Stunden durch Abbrennen von kohlenstoffreichen Ablagerungen befreit und hatte nach einer reduktiven Behandlung mit Wasserstoff bei 350°C die Aktivität eines frischen Kontaktes.

Beispiel 3

In der in Beispiel 2 beschriebenen Apparatur wurde unter den gleichen Bedingungen N-Cyclohexyliden-3-methyl-anilin umgesetzt. Kondensierte man das Reaktionsprodukt und analysierte es gaschromatographisch, so zeigte es die folgenden Zeitabhängigkeiten in der Zusammensetzung:

| Betriebsstunden | 100 | 200 | 1000 | 2000 |
|---|---|---|---|---|
| 3-Methyl-diphenylamin | 95,4 | 95,5 | 95,9 | 95,1 |
| N-Cyclohexyl-3-methyl-anilin | 0,25 | 0,1 | 0,08 | 0,18 |
| N-Cyclohexyliden-3-methyl-anilin | 0,05 | - | - | 0,08 |
| 3-Methyl-anilin | 2,3 | 2,1 | 1,9 | 2,1 |
| Benzol | 1,2 | 1,4 | 1,3 | 1,5 |
| Hochsieder *) | 0,8 | 0,9 | 0,8 | 1,0 |

*) Methylcarbazol, Methyl-phenyl'-diphenylamin, Methyl-phenyl'-carbazol

**Patentansprüche**

1. Verfahren zur Herstellung von Diphenylaminen der Formel

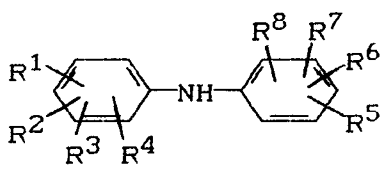

in der

$R^1$ für Wasserstoff, $C_1$-$C_6$-Alkyl, $C_1$-$C_2$-Alkoxy, $C_3$-$C_6$-Cycloalkyl, Benzyl oder Aryl steht,

$R^2$ für Wasserstoff, $C_1$-$C_6$-Alkyl oder $C_1$-$C_2$-Alkoxy steht,

$R^5$ Wasserstoff, $C_1$-$C_6$-Alkyl, $C_1$-$C_6$-Alkoxy, $C_3$-$C_6$-Cycloalkyl, Benzyl, Aryl, Hydroxy, Amino, $C_1$-$C_6$-Alkylamino, Di-$C_1$-$C_6$-alkylamino, Aryloxy oder Arylamino bedeutet,

$R^6$ für Wasserstoff, $C_1$-$C_6$-Alkyl, $C_1$-$C_6$-Alkoxy, Hydroxy, Amino oder $C_1$-$C_6$-Alkylamino steht und

$R^3$, $R^4$, $R^7$ und $R^8$ unabhängig voneinander Wasserstoff oder $C_1$-$C_2$-Alkyl bedeuten,

wobei Aryl für Phenyl oder für ein in 2-, 3- oder 4-Stellung gebundenes 5- oder 6-gliedriges Heteroaryl mit 1 oder 2 Heteroatomen aus der Gruppe N, O und S steht,

dadurch gekennzeichnet, daß man N-Cyclohexyliden-aniline der Formel

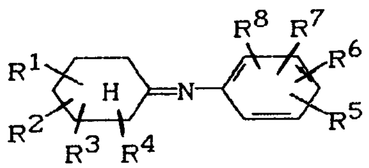 ,

in der die Reste

$R^1$ bis $R^8$ die obige Bedeutung haben,

bei 250-500°C und einem Druck von 10 mbar bis 100 bar an einem Katalysator dehydriert, der einen Rhodiumgehalt ohne weitere Platinmetalle von 0,05-5 Gew.-%, bevorzugt 0,05-4 Gew.-%, besonders bevorzugt 0,1-3 Gew.-% aufweist, und der weiterhin Zusätze von 1-6 Gew.-% eines Alkalimetallhydroxids und 1-6 Gew.-% eines Alkalimetallsulfats enthält, wobei alle Prozentsätze auf das Gesamtgewicht des Katalysators bezogen sind.

2. Verfahren nach Anspruch 1, dadurch gekennzeichnet, daß der Träger des Katalysators $Al_2O_3$ oder ein Aluminiumspinell ist.

3. Verfahren nach Anspruch 1, dadurch gekennzeichnet, daß der Träger des Katalysators ein mit Chrom und Mangan behandeltes Aluminiumoxid oder Aluminiumspinell ist,

4. Verfahren nach Anspruch 1, dadurch gekennzeichnet, daß das Träger des Katalysators eine Oberfläche größer 100 $m^2/g$ besitzt.

5. Verfahren nach Anspruch 1, dadurch gekennzeichnet, daß in einem Temperaturbereich von 280-450°C, bevorzugt von 300-425°C gearbeitet wird.

6. Verfahren nach Anspruch 1, dadurch gekennzeichnet, daß in einem Druckbereich von 100 mbar bis 2 bar, bevorzugt von 0,9 bis 1,2 bar gearbeitet wird.

7. Verfahren nach Anspruch 1, dadurch gekennzeichnet, daß ein gasförmiges Verdünnungsmittel, wie Wasserstoff, Methan, Ethan, Stickstoff, Erdgas, Helium oder Argon, bevorzugt Methan, Erdgas, Wasserstoff oder Stickstoff, ganz besonders bevorzugt Stickstoff oder Erdgas benutzt wird.

8. Verfahren nach Anspruch 1, dadurch gekennzeichnet, daß die Menge des gasförmigen Verdünnungsmittels oder Trägergases 0,1-20, bevorzugt 1-10, ganz besonders bevorzugt 4-8 Mol je Mol Ausgangsverbindung beträgt.

9. Verfahren nach Anspruch 1, dadurch gekennzeichnet, daß die Katalysatorbelastung bei 0,01-1, bevorzugt bei 0,1-0,8, besonders bevorzugt bei 0,3-0,5 g/ml/ Stunde liegt.

## Claims

1. Process for preparing diphenylamines of the formula

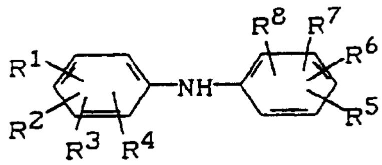

in which

| | |
|---|---|
| $R^1$ | represents hydrogen, $C_1$-$C_6$-alkyl, $C_1$-$C_2$-alkoxy, $C_3$-$C_6$-cycloalkyl, benzyl or aryl, |
| $R^2$ | represents hydrogen, $C_1$-$C_6$-alkyl or $C_1$-$C_2$-alkoxy, |
| $R^5$ | represents hydrogen, $C_1$-$C_6$-alkyl, $C_1$-$C_6$-alkoxy, $C_3$-$C_6$-cycloalkyl, benzyl, aryl, hydroxy, amino, $C_1$-$C_6$-alkylamino, di-$C_1$-$C_6$-alkylamino, aryloxy or arylamino, |
| $R^6$ | represents hydrogen, $C_1$-$C_6$-alkyl, $C_1$-$C_6$-alkoxy, hydroxy, amino or $C_1$-$C_6$-alkylamino and |
| $R^3$, $R^4$, $R^7$ and $R^8$, | independently of one another, represent hydrogen or $C_1$-$C_2$-alkyl, |

where aryl represents phenyl or represents a 5- or 6-membered heteroaryl attached via position 2, 3 or 4 and containing 1 or 2 heteroatoms selected from the group consisting of N, O and S, characterized in that N-cyclohexylideneanilines of the formula

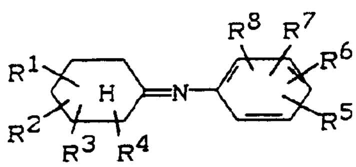

,

in which the radicals
$R^1$ to $R^8$ are as specified above,
are dehydrogenated at 250-500°C and a pressure from 10 mbar to 100 bar over a catalyst which has a rhodium content without further platinum metals of 0.05-5 % by weight, preferably 0.05-4 % by weight, particularly preferably 0.1-3 % by weight, and which further contains additions of 1-6 % by weight of an alkali metal hydroxide and 1-6 % by weight of an alkali metal sulphate, all percentages being based on the total weight of the catalyst.

2. Process according to Claim 1, characterized in that the catalyst support is $Al_2O_3$ or an aluminium spinel.

3. Process according to Claim 1, characterized in that the catalyst support is an aluminium oxide or aluminium spinel treated with chromium and manganese.

4. Process according to Claim 1, characterized in that the catalyst support possesses a surface area of greater than 100 $m^2/g$.

5. Process according to Claim 1, characterized in that it is carried out in a temperature range of 280-450°C, preferably of 300-425°C.

6. Process according to Claim 1, characterized in that it is carried out in a pressure range from 100 mbar to 2 bar, preferably from 0.9 to 1.2 bar.

7. Process according to Claim 1, characterized in that use is made of a gaseous diluent such as hydrogen, methane, ethane, nitrogen, natural gas, helium or argon, preferably methane, natural gas, hydrogen or nitrogen, very particularly preferably nitrogen or natural gas.

8. Process according to Claim 1, characterized in that the amount of the gaseous diluent or carrier gas is 0.1-20, preferably 1-10, very particularly preferably 4-8, mol per mole of starting compound.

9. Process according to Claim 1, characterized in that the space velocity over the catalyst is 0.01-1, preferably 0.1-0,8, particularly preferably 0.3-0.5 g/ml/hour.

## Revendications

1. Procédé de production de diphénylamines de formule

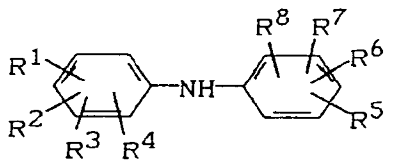

dans laquelle

| | |
|---|---|
| $R^1$ | représente de l'hydrogène, un groupe alkyle en $C_1$ à $C_6$, alkoxy en $C_1$ ou $C_2$, cycloalkyle en $C_3$ à $C_6$, benzyle ou aryle, |
| $R^2$ | est de l'hydrogène, un groupe alkyle en $C_1$ à $C_6$ ou alkoxy en $C_1$ ou $C_2$, |
| $R^5$ | est de l'hydrogène, un groupe alkyle en $C_1$ à $C_6$, alkoxy en $C_1$ à $C_6$, cycloalkyle en $C_3$ à $C_6$, benzyle, aryle, hydroxy, amino, alkylamino en $C_1$ à $C_6$, di-(alkyle en $C_1$ à $C_6$)-amino, aryloxy ou arylamino, |
| $R^6$ | représente de l'hydrogène, un groupe alkyle en $C_1$ à $C_6$, alkoxy en $C_1$ à $C_6$, hydroxy, amino ou alkylamino en $C_1$ à $C_6$ et |
| $R^3$, $R^4$, $R^7$ et $R^8$ | représentent, indépendamment les uns des autres, de l'hydrogène ou un groupe alkyle en $C_1$ ou $C_2$, |

le terme aryle désignant un groupe phényle ou un groupe hétéro-aryle pentagonal ou hexagonal lié en position 2, 3 ou 4 et portant 1 ou 2 hétéro-atomes du groupe N, O et S,

caractérisés en ce qu'on déshydrogène des N-cyclohexylidène-anilines de formule

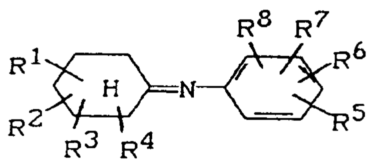

,

dans laquelle les restes $R^1$ à $R^8$ ont la définition indiquée ci-dessus,

à 250-500°C et sous une pression allant de 10 mbars à 100 bars sur un catalyseur qui présente une teneur en rhodium, sans autres métaux du groupe du platine, de 0,05 à 5 % en poids, de préférence de 0,05 à 4 % en poids, notamment de 0,1 à 3 % en poids et qui contient en outre des additions de 1 à 6 % en poids d'un hydroxyde de métal alcalin et de 1 à 6 % en poids d'un sulfate de métal alcalin, tous les pourcentages se rapportant au poids total du catalyseur.

2. Procédé suivant la revendication 1, caractérisé en ce que le support du catalyseur consiste en $Al_2O_3$ ou en un spinelle d'aluminium.

3. Procédé suivant la revendication 1, caractérisé en ce que le support du catalyseur est un oxyde d'aluminium ou un spinelle d'aluminium traité au chrome et au manganèse.

4. Procédé suivant la revendication 1, caractérisé en ce que le support du catalyseur a une surface spécifique supérieure à 100 $m^2/g$.

5. Procédé suivant la revendication 1, caractérisé en ce qu'on opère dans une plage de températures de 280 à 450°C, de préférence de 300 à 425°C.

6. Procédé suivant la revendication 1, caractérisé en ce qu'on opère dans une plage de pressions de 100

mbars à 2 bars, de préférence de 0,9 à 1,2 bar.

7. Procédé suivant la revendication 1, caractérisé en ce qu'on utilise un diluant gazeux tel que l'hydrogène, le méthane, l'éthane, l'azote, le gaz naturel, l'hélium ou l'argon, de préférence le méthane, le gaz naturel, l'hydrogène ou l'azote, notamment l'azote ou le gaz naturel.

8. Procédé suivant la revendication 1, caractérisé en ce que la quantité de diluant gazeux ou de véhicule gazeux est de 0,1 à 20, de préférence de 1 à 10, notamment de 4 à 8 moles par mole de composé de départ.

9. Procédé suivant la revendication 1, caractérisé en ce que la charge du catalyseur est de 0,01 à 1, de préférence de 0,1 à 0,8, notamment de 0,3 à 0,5 g/ml/heure.